Europäisches Patentamt

European Patent Office

Office européen des brevets

(11) Publication number: **0 098 122 B1**

# EUROPEAN PATENT SPECIFICATION

(45) Date of publication of patent specification: **31.05.89**

(51) Int. Cl.⁴: **C 12 P 13/24**

(21) Application number: **83303661.9**

(22) Date of filing: **24.06.83**

(54) Processes for producing L-proline by fermentation.

(30) Priority: **24.06.82 JP 108834/82**
**13.12.82 JP 218077/82**

(43) Date of publication of application:
**11.01.84 Bulletin 84/02**

(45) Publication of the grant of the patent:
**31.05.89 Bulletin 89/22**

(84) Designated Contracting States:
**DE FR GB IT**

(56) References cited:
**EP-A-0 054 311**
**US-A-3 819 483**
**US-A-4 389 483**
**US-A-4 389 483**
**CHEMICAL ABSTRACTS, vol. 97, no. 13,**
**September 1982, page 483, no. 108538y,**
**Columbus, Ohio, US; & JP - A - 82 65 198**
**(AJINOMOTO CO., INC.) 20-04-1982**

The file contains technical information
submitted after the application was filed and
not included in this specification

(73) Proprietor: **KYOWA HAKKO KOGYO CO., LTD.**
**Ohtemachi Bldg., 6-1 Ohtemachi l-chome**
**Chiyoda-ku Tokyo 100 (JP)**

(72) Inventor: **Nakanishi, Toshihide**
**2-3-205 Kyowa-cho**
**Hofu-shi Yamaguchi-ken (JP)**
Inventor: **Yonekura, Hideaki**
**2-10-104 Kyowa-cho**
**Hofu-shi Yamaguchi-ken (JP)**
Inventor: **Hattori, Kiyoji**
**2-12-106 Kyowa-cho**
**Hofu-shi Yamaguchi-ken (JP)**
Inventor: **Hirao, Toshihiko**
**2-9-306 Kyowa-cho**
**Hofu-shi Yamaguchi-ken (JP)**
Inventor: **Azuma, Tomoki**
**1-2 Kyowa-cho**
**Hofu-shi Yamaguchi-ken (JP)**

(74) Representative: **Lambert, Hugh Richmond et al**
**D. YOUNG & CO. 10 Staple Inn**
**London, WC1V 7RD (GB)**

Courier Press, Leamington Spa, England.

EP 0 098 122 B1

(56) References cited:
**CHEMICAL ABSTRACTS, vol. 94, no. 6, March
1981, page 565, no. 82175p, Columbus, Ohio,
US; & JP - A - 80 148 094 (KYOWA HAKKO
KOGYO CO., LTD.) 18-11-1980**

**CHEMICAL ABSTRACTS, vol. 82, no. 19, May
1975, page 409, no. 123315g, Columbus, Ohio,
US; & JP - A - 74 100 291 (KYOWA HAKKO
KOGYO CO., LTD.) 21-09-1974**

**CHEMICAL ABSTRACTS, vol. 87, no. 25,
December 1977, page 543, no. 199202s,
Columbus, Ohio, US; & JP - A - 77 96 793
(KYOWA HAKKO KOGYO CO., LTD.) 13-08-1977**

**Description**

The present invention relates to the production of L-proline by fermentation.

L-proline is an important amino acid which is commercially useful as a food additive.

It is already known that L-proline can be produced by fermentation using microorganisms of the genera *Corynebacterium* and *Brevibacterium*. However, the production yields of the known processes are comparatively low from a commercial point of view. Thus, a need exists for processes for producing L-proline in high yield and at low cost.

To this end, it has now been found that L-proline productivity of an L-proline producing microorganism belonging to the genus *Corynebacterium* or *Brevibacterium* is greatly improved if the microorganism is endowed with a resistance to a purine or pyrimidine analog. Further, it has been found that L-proline is produced in good yield by culturing the mutant in the presence of a specified amount of $NH_4^+$ ion.

In accordance with the invention, therefore, L-proline is produced by culturing an L-proline producing microorganism belonging to the genus *Corynebacterium* or *Brevibacterium* in a nutrient medium until L-proline is accumulated in the culture liquor and thereafter recovering said L-proline, wherein a mutant strain is used which with respect to the parent strains has, or has been endowed with, a resistance to at least one purine or pyrimidine analog. In a preferred embodiment, L-proline is produced by culturing the mutant strain in a culture medium containing from 0.05 to 0.5 M of the $NH_4^+$ ion.

The purine analog or pyrimidine analog resistant mutants used in this invention can be obtained by endowing an L-proline-producing strain of a microorganism of the genus *Corynebacterium* or *Brevibacterium* with a resistance to a purine analog or a pyrimidine analog or both, or by endowing a microorganism strain already having a resistance to a purine analog or a pyrimidine analog or both with the ability to produce L-proline.

The purine analog to which the mutant has resistance may be any of the following: 6-mercaptoguanine, 8-azaguanine, 2-fluoroadenine, tubercidin, 6-methylpurine (hereinafter referred to as "6MP"), 8-azaxanthine, 8-azaadenine, 8-mercaptoguanosine, 6-mercaptoguanosine (hereinafter referred to as "6MG"), 2-aminopurine, 2-amino-6-mercaptopurine, decoyinin and psicofuranine. Likewise, the pyrimidine analog to which the mutant has resistance may be any of the following: 5-bromouracil (hereinafter referred to as "5BU"), 6-azauracil (hereinafter referred to as "6AU"), 5-fluorouracil 5-bromo-2-deoxyuridine, 2-thiouracil, 6-methyl-2-thiouracil and amicetin.

Moreover, mutant strains having other properties such as various nutrient requirements, drug resistance, drug sensitivity and drug dependence in addition to the above-mentioned resistance may be employed.

Suitably mutated strains for use in this invention can be obtained by culturing and screening suitable parent strains in a nutrient medium containing a pyrimidine or purine analog and selecting the strain or strains having the ability to produce L-proline in greater yields than the parent strain. For example, a selected parent strain in suspension in a 0.1 N trismaleate buffer solution (pH 6.0) in a concentration of $10^8$ cells/ml, is treated with a suitable analog e.g. N-methyl-N'-nitro-N-nitrosoguanidine to make a final concentration of 0.2 mg/ml. The suspension is allowed to stand at room temperature for 30 minutes and then smeared on a flat agar plate of minimal medium of the following composition containing 0.4 mg/ml of 6MG, 0.2 mg/ml of 6MP, 0.1 mg/ml of 5BU and 0.1 mg/ml of 6AU. The desired mutants can then be selected from the growing colonies.

The composition of the flat agar plate of minimal medium is as follows:

10 g/l of glucose, 4 g/l of ammonium chloride, 1 g/l of $KH_2PO_4$, 3 g/l of $K_2HPO_4$, 0.4 g/l of $MgSO_4 \cdot 7H_2O$, 0.01 g/l of $FeSO_4 \cdot 7H_2O$, 0.01 g/l of $MnSO_4 \cdot 4H_2O$, 2 g/l of urea, 50 µg/l of biotin and 20 g/l of agar. pH: 7.2.

Examples of preferred strains used in the present invention are as follows:

TABLE 1

| No. | Strain | FERM P-No. | Property of mutant | Parent strain[2] |
|---|---|---|---|---|
| 1 | *Corynebacterium glutamicum* H-3334 | 6823 | 6 MG$^R$ | 1 |
| 2 | *Corynebacterium acetophilum* H-3336 | 6824 | 6MP$^R$ | 2 |
| 3 | *Corynebacterium acetophilum* H-3337 | 6825 | 5 BU$^R$ | 2 |
| 4 | *Corynebacterium acetoacidphilum* H-3338 | 6826 | 6 AU$^R$ | 3 |
| 5 | *Brevibacterium lactofermentum* H-3356 | 6827 | 5 BU$^R$ | 4 |
| 6 | *Brevibacterium lactofermentum* H-3357 | 6828 | 6 MP$^R$ | 4 |

[2] [1] *Corynebacterium glutamicun* ATCC 13032      [3] *Corynebacterium acetoacidphilum* ATCC 13870
[2] *Corynebacterium acetophilum* FERM-P 4045      [4] *Brevibacterium lactofermentum* ATCC 13869

The strains listed in Table 1 are clearly discriminated from the parent strain, as shown in Table 2.

TABLE 2

| Additive (µg/ml) | No addition | 6MG | | 6MP | | 6AU | | | 5BU | |
|---|---|---|---|---|---|---|---|---|---|---|
| | | 1000 | 200 | 200 | 50 | 100 | 50 | 10 | 1000 | 200 |
| ATCC 13032 | ++ | − | ± | | | | | | | |
| H-3334 (FERM P-6823) | ++ | ++ | ++ | | | | | | | |
| FERM-P 4045 | ++ | | | − | ± | | | | − | + |
| H-3336 (FERM P-6824) | ++ | | | ++ | ++ | | | | | |
| H-3337 (FERM P-6825) | ++ | | | | | | | | ++ | ++ |
| ATCC 13870 | ++ | | | | | − | ± | + | | |
| H-3338 (FERM P-6826) | ++ | | | | | ++ | ++ | ++ | | |
| ATCC-13869 | ++ | | | − | ± | | | | − | + |
| H-3356 (FERM P-6827) | ++ | | | | | | | | | |
| H-3357 (FERM P-6828) | ++ | | | ++ | ++ | | | | ++ | ++ |

(++: sufficient growth; +: growth to some extent; ±: a little growth; −: no growth)

The aforesaid mutants H-3334, H-3336, H-3337, H-3338, H-3356 and H-3357 were deposited with the Fermentation Research Institute, The Agency of Industrial Science and Technology Japan as FERM-P numbers shown in Table 1, on 9th December, 1982.

All of the mutants are directly transferred from the Fermentation Research Institute described above to Agricultural Research Cultural Collection (NRRL) and are deposited under the terms of the Budapest Treaty.

As the medium used for the culturing process of the present invention, either a natural medium or synthetic medium may be employed as long as it contains an assimilable carbon source, a nitrogen source and the inorganic materials and other nutrients which may be required by the specific mutant used.

A suitable carbon sources there may be used carbohydrates such as sucrose, fructose, glucose, maltose, mannose, starch, starch hydrolyzate and molasses; sugar alcohols such as glycerine and sorbitol; organic acids such as formic acid, acetic acid, lactic acid, fumaric acid and malic acid; and alcohols such as ethanol and methanol. These carbon sources may be employed either alone or as mixtures at various weight ratios. The total amount may be initially supplied in the medium or may be supplied by incremental addition.

As suitable nitrogen sources there may be used inorganic and organic ammonium salts such as ammonium chloride, ammonia, ammonium sulfate, ammonium carbonate, ammonium acetate, ammonium nitrate and ammonium phosphate, urea; natural nitrogen containing substances such as peptone, meat extract, corn steep liquor, casein hydrolyzate and soybean meal hydrolyzate. Inorganic materials which may be required include potassium dihydrogen phosphate, dipotassium hydrogen phosphate, magnesium sulfate, sodium chloride, ferrous sulfate, manganese sulfate and calcium carbonate.

When the strain used requires nutrients such as amino acids, nucleic acids and vitamins, it is, of course, also necessary to include appropriate amounts of these substances in the medium. In some cases, such nutrients may be supplied by another medium component and thus specific supplementation is not required.

Culturing is generally carried out at 20—40°C, under aerobic conditions, for example, by shaking culture or aeration culture until a recoverable quantity of L-proline is produced in the culture liquor, usually within 1—8 days. After the completion of culturing, the product L-proline may be recovered from the culture liquor by conventional methods such as by ion exchange resin treatment.

The process of the invention is illustrated by the following representative examples.

4

**Example 1**

Each strain identified in Table 3 is inoculated in a 250 ml-Erlenmeyer flask containing 30 ml of a sterile seed medium (pH 7.2) comprising 10 g/l glucose, 5 g/l meat extract, 10 g/l peptone and 3 g/l yeast extract, and cultured with shaking (220 rpm) at 30°C for 24 hours. Then, 30 ml of the resulting seed culture liquor is put into a 2 l-Erlenmeyer flask provided with baffle containing 300 ml of a sterile fermentation medium (pH 7.4) comprising 100 g/l glucose, 3 g/l $KH_2PO_4$, 0.5 g/l $MgSO_4 \cdot 7H_2O$, 10 g/l ammonium sulfate, 10 mg/l $FeSO_4 \cdot 7H_2O$, 10 mg/l nicotinic acid, 100 µg/l thiamine hydrochloride, 100 µg/l biotin, 20 g/l corn steep liquor, 20 g/l sodium L-glutamate and 30 g/l $CaCO_3$, and cultured with shaking (220 rpm) at 32°C for 4 days.

Amounts of L-proline accumulated in the culture liquors are given in Table 3.

TABLE 3

| Strain | L-proline (g/l) |
|---|---|
| ATCC 13032 | 0.5 |
| H-3334 (FERM P-6823) | 18.5 |
| FERM-P 4045 | 24.0 |
| H-3336 (FERM P-6824) | 38.0 |
| H-3337 (FERM P-6825) | 37.0 |
| ATCC 13870 | 1.0 |
| H-3338 (FERM P-6826) | 38.5 |
| ATCC 13869 | 0.5 |
| H-3356 (FERM P-6827) | 25.5 |
| HG-3357 (FERM P-6828) | 27.0 |

**Example 2**

In this Example, using each strain identified in Table 4 as a seed strain and the same seed culture mediums as obtained in Example 1, the resulting culture liquor is subjected to a main fermentation in the same manner and in the same culture medium as in Example 1, except that the concentration of ammonium sulfate to be contained therein is changed to those shown in Table 4.

Amounts of L-proline accumulated in the culture liquors are given in Table 4.

TABLE 4

| Ammonium sulfate ($NH_4^+$ concentration M) | H-3338 (FERM P-6826) | H-3357 (FERM P-6828) | FERM P-4045 |
|---|---|---|---|
| 0 | 15.0 | 11.0 | 2.0 |
| 0.02 | 21.5 | 17.5 | 8.0 |
| 0.05 | 36.0 | 25.0 | 13.0 |
| 0.2 | 38.0 | 27.0 | 21.0 |
| 0.5 | 37.0 | 26.5 | 33.0 |
| 1.0 | 32.0 | 20.0 | 35.5 |
| 2.0 | 19.0 | 12.0 | 29.0 |

**Example 3**

Strain H-3336 (FERM P-6824) or H-3338 (FERM P-6826) is inoculated in a 2 l-Erlenmeyer flask provided

with baffle containing 200 ml of a sterile seed medium comprising the following components, and cultured with shaking (220 rpm) at 30°C for 24 hours.

Seed culture medium

| | |
|---|---|
| Sucrose | 60 g/l |
| $KH_2PO_4$ | 2 g/l |
| $MgSO_4 \cdot 7H_2O$ | 0.5 g/l |
| $FeSO_4 \cdot 7H_2O$ | 10 mg/l |
| $MnSO_4 \cdot 4H_2O$ | 10 mg/l |
| Corn steep liquor | 10 g/l |
| Thiamine hydrochloride | 100 µg/l |
| Biotin | 100 µg/l |
| Urea | 3 g/l |

pH 7.4

Then, 100 ml of the seed culture liquor is put into a 2 l-jar fermenter containing 700 ml of a sterile fermentation medium comprising the following components, and cultured with aeration-stirring at 32°C. In the course of culturing, pH of the culture liquor is adjusted to 7.0 by using acetic acid solution as a continuous feed.

Fermentation medium

| | |
|---|---|
| Sucrose | 20 g/l |
| Ammonium acetate | 7 g/l |
| $MgSO_4 \cdot 7H_2O$ | 0.6 g/l |
| $KH_2PO_4$ | 4 g/l |
| Ammonium sulfate | 5 g/l |
| $FeSO_4 \cdot 7H_2O$ | 10 mg/l |
| $MnSO_4 \cdot 4H_2O$ | 10 mg/l |
| $CuSO_4 \cdot 5H_2O$ | 1 mg/l |
| Thiamine hydrochloride | 100 µg/l |
| Biotin | 100 µg/l |

pH 7.4

When culturing is continued for 72 hours, acetic acid is consumed at a rate of 15% to the starting culture liquor, and amounts of L-proline accumulated in the culture liquors of H-3336 (FERM P-6824) and H-3338 (FERM P-6826) are 45 g/l and 43 g/l, respectively. Amounts of L-proline, in case where corresponding parent strains FERM P-4045 and ATCC 13870 are cultured at the same time under the same conditions as a control, are 28.5 g/l and 0.7 g/l.

## Claims

1. A process for producing L-proline by fermentation which comprises culturing an L-proline producing microorganism belonging to the genus *Corynebacterium* or *Brevibacterium* in a nutrient medium until L-proline is accumulated in the culture liquor and thereafter recovering said L-proline therefrom, characterized in that the microorganism used is a mutant strain which with respect to the parent strain has or has been endowed with a resistance to at least one purine analog and/or at least one pyrimidine analog.

2. A process according to claim 1, wherein said microorganism is a mutant strain of *Corynebacterium glutamicum* or *Brevibacterium lactofermentum*.

3. A process according to claim 1 or 2, wherein the mutant microorganism has resistance to one or more of the following purine analogs: 6-mercaptoguanine, 8-azaguanine, 2-fluoroadenine, tubercidin, 6-methylpurine, 8-azaxanthine, 8-azaadenine, 8-mercaptoguanosine, 6-mercaptoguanosine, 2-aminopurine, 2-amino-6-mercaptopurine, decoyinin or psicofuranine and/or resistance to one or more of the following pyrimidine analogs: 5-bromouracil, 6-azauracil, 5-fluorouracil, 5-bromo-2-deoxyuridine, 2-thiouracil, 6-methyl-2-thiouracil or amicetin.

4. A process according to any one of claims 1—3, wherein said culturing is conducted at 20 to 40°C for 1 to 8 days.

5. A process according to any one of claims 1—4, wherein the culture medium contains from 0.05 to 0.5 M or $NH_4^+$ ion.

6. A process according to any one of the preceding claims, wherein said mutant microorganism strain is *Corynebacterium glutamicum* H-3334 (FERM P-6823), *Corynebacterium acetophilum* H-3336 (FERM P-6824), *Corynebacterium acetophilum* H-3337 (FERM P-6825), *Corynebacterium acetoacidphilum* H-3338 (FERM P-6826), *Brevibacterium lactofermentum* H-3356 (FERM P-6827), or *Brevibacterium lactofermentum* H-3357 (FERM P-6828).

**Patentansprüche**

1. Verfahren zur Herstellung von L-Prolin durch Fermentation, umfassend die Züchtung eines L-Prolin erzeugenden, zur Gattung *Corynebacterium* oder *Brevibacterium* gehörenden Mikroorganismus in einem Nährmedium, bis L-Prolin in der Nährflüssigkeit angesammelt ist und dann Gewinnung des L-Prolins daraus, dadurch gekennzeichnet, daß der verwendete Mikroorganismus ein Mutantenstamm ist, der im Hinblick auf den Elternstamm Resistenz gegen mindestens ein Purin-Analoges und/oder mindestens ein Pyrimidin-Analoges aufweist oder diese erhalten hat.

2. Verfahren nach Anspruch 1, in dem der Mikroorganismus ein Mutantenstamm von *Corynebacterium glutamicum* oder *Brevibacterium lactofermentum* ist.

3. Verfahren nach Anspruch 1 oder 2, in dem der Mutanten-Mikroorganismus Resistenz gegen eines oder mehrere der folgenden Purin-Analoge: 6-Mercaptoguanin, 8-Azaguanin, 2-Fluoradenin, Tubercidin, 6-Methylpurin, 8-Azaxanthin, 8-Azaadenin, 8-Mercaptoguanosin, 6-Mercaptoguanosin, 2-Aminopurin, 2-Amino-6-mercatopurin, Decoyinin oder Psicofuranin und/oder Resistenz gegen eines oder mehrere der folgenden Pyrimidin-Analoge aufweist: 5-Bromuracil, 6-Azauracil, 5-Fluoruracil, 5-Brom-2-deoxyuridin, 2-Thiouracil, 6-Methyl-2-thiouracil oder Amicetin.

4. Verfahren nach einem der Ansprüche 1 bis 3, in dem die Züchtung bei 20 bis 40°C 1 bis 8 Tage durchgeführt wird.

5. Verfahren nach einem der Ansprüche 1 bis 4, in dem das Kulturmedium 0,05 bis 0,5 M $NH_4^+$-Ionen enthält. ·

6. Verfahren nach einem der vorangehenden Ansprüche, in dem der Mutanten-Mikroorganismusstamm *Corynebacterium glutamicum* H-3334 (FERM P-6823), *Corynebacterium acetophilum* H-3336 (FERM P-6824), *Corynebacterium acetophilum* H-3337 (FERM P-6825), *Corynebacterium acetoacidphilum* H-3338 (FERM P-6826), *Brevibacterium lactofermentum* H-3356 (FERM P-6827) oder *Brevibacterium lactofermentum* H-3357 (FERM P-6828) ist.

**Revendications**

1. Procédé de production de L-proline par fermentation dans lequel on cultive, dans un milieu nutritif, un micro-organisme producteur de L-proline appartenant au genre *Corynebacterium* ou *Brevibacterium* jusqu'à ce que la L-proline soit accumulée dans la liqueur de culture et récupère après ladite L-proline à partir de la liqueur, caractérisé en ce que le microorganisme utilisé est une souche mutante qui, par rapport à la souche mère, possède une résistance à au moins un analogue de la purine et/ou au moins un analogue de la pyrimidine ou a été doté d'une telle résistance.

2. Procédé selon la revendication 1, dans lequel ledit micro-organisme est une souche mutante de *Corynebacterium Glutamicum* ou de *Brevibacterium lactofermentum.*

3. Procédé selon la revendication 1 ou 2, dans lequel le micro-organisme mutant possède une résistance à un ou plusieurs des analogues suivants de la purine: 6-mercaptoguanine, 8-azaguanine, 2-fluoroadénine, tubercidine, 6-méthylpurine, 8-azaxanthine, 8-azaadénine, 8-mercaptoguanosine, 6-mercaptoguanosine, 2-aminopurine, 2-amino-6-mercaptopurine, décoyinine ou psicofuranine, et/ou une résistance à un ou plusieurs des analogues suivants de la pyrimidine: 5-bromouracile, 6-azauracile, 5-fluorouracile, 5-bromo-2-désoxyuridine, 2-thiouracile, 6-méthyl-2-thiouracile ou amicétine.

4. Procédé selon l'une quelconque des revendications 1 à 3, dans lequel la culture est effectuée à une température de 20 à 40°C pendant 1 à 8 jours.

5. Procédé selon l'une quelconque des revendications 1 à 4, dans lequel le milieu de culture contient des ions $NH_4^+$ à une concentration 0,05 à 0,5 M.

6. Procédé selon l'une quelconque des revendications précédentes, dans lequel ladite souche de micro-organisme mutante est *Corynebacterium glutamicum* H-3334 (FERM P-6823), *Corynebacterium acetophilum* H-3336 (FERM P-6824), *Corynebacterium acetophilum* H-3337 (FERM P-6825), *Corynebacterium acetoacidphilum* H-3338 (FERM P-6826), *Brevibacterium lactofermentum* H-3356 (FERM P-6827) ou *Brevibacterium lactofermentum* H-3357 (FERM P-6828).